# EUROPÄISCHE PATENTSCHRIFT

(11) **EP 1 537 829 B1**
(45) Veröffentlichungstag und Bekanntmachung des Hinweises auf die Patenterteilung: **03.12.2008**
(21) Anmeldenummer: 04025438.5
(22) Anmeldetag: 26.10.2004
(51) Int. Cl.: A61B 17/28

(54) **Medizinisches Instrument mit Instrumenteneinsatz und Mittel zur Blockierung des beweglichen Griffteils**
Medical device comprising instrument insert and means for blocking the movement of the handle
Dispositif médical comprenant insert et moyen pour le blocage de la manche

(30) Priorität: 06.12.2003 DE 10357105
(43) Veröffentlichungstag der Anmeldung: 08.06.2005
(73) Patentinhaber: Richard Wolf GmbH, 75438 Knittlingen (DE)
(72) Erfinder: Knodel, Frank, Dipl.-Ing., 75438 Knittlingen (DE); Prestel, Stephan, 76287 Rheinstetten-Mörsch (DE); Wagner, Carl-Sebastian, 75015 Bretten (DE); Bartolic, Josef, 76229 Karlsruhe-Grötzingen (DE)
(74) Vertreter: Hemmer, Arnd

(56) Entgegenhaltungen:
- DE-A1- 4 341 736
- US-B1- 6 358 267

## Beschreibung

Die Erfindung betrifft ein medizinisches Instrument und insbesondere eine medizinische Zange.

Beispielsweise aus DE 198 09 120 Cl ist ein chirurgisches Rohrschaftinstrument bekannt, welches ein Griffteil und einen mit diesem lösbar verbindbaren Schaft aufweist. Im Schaft ist eine Betätigungsstange bewegbar angeordnet, um Teile, beispielsweise ein Zangenmaul, am distalen Ende des Schaftes über einen beweglichen Handgriff an dem Griffteil bewegen zu können. Sowohl der Schaft als auch die Betätigungsstange werden mit dem Griffteil bzw. einer in diesem angeordneten Betätigungsstange über Kugelrastverbindungen verbunden. Zur vollständigen Verbindung des Schaftes und der Betätigungsstange mit dem Griffteil müssen somit zwei Kugelrastverbindungen verrastet werden. Das Zusammensetzen ist daher umständlich, und es ist für den Anwender nicht direkt erkennbar, ob beide Verbindungen sicher eingerastet sind und die Funktionsfähigkeit des Instrumentes sichergestellt ist.

Es ist daher Aufgabe der Erfindung, ein verbessertes medizinisches Instrument bereitzustellen, welches eine einfachere Verbindung eines Instrumenteneinsatzes mit einer Instrumentenhandhabe ermöglicht und den Benutzer die Funktionsbereitschaft des Instrumentes leicht erkennen lässt.

Diese Aufgabe wird durch ein medizinisches Instrument mit den im Anspruch 1 angegebenen Merkmalen gelöst. Bevorzugte Ausführungsformen ergeben sich aus den Unteransprüchen.

Das erfindungsgemäße medizinische Instrument, beispielsweise eine medizinische Zange, weist einen Instrumenteneinsatz und eine Instrumentenhandhabe auf, welche lösbar miteinander verbindbar sind. Die Lösbarkeit ermöglicht den Austausch der Instrumenteneinsätze, so dass mit ein und derselben Instrumentenhandhabe verschiedene Instrumenteneinsätze kombiniert werden können. Ferner kann der Instrumenteneinsatz zur Wartung oder Reinigung leicht von der Instrumentenhandhabe getrennt werden, welche dann mit einem anderen Instrumenteneinsatz weiter verwendet werden kann. Im Inneren des Instrumenteneinsatzes ist eine Betätigungsstange zur Bewegung von Elementen am distalen Ende des Instrumenteneinsatzes, beispielsweise eines Zangenmauls, angeordnet. Die Betätigungsstange ist über eine Rastverbindung lösbar mit einem beweglichen Griffteil der Instrumentenhandhabe verbindbar. Im verbundenen Zustand überträgt die Betätigungsstange dann eine Bewegung des Griffteils auf die zu bewegenden Elemente am distalen Ende des Instrumenteneinsatzes.

Erfindungsgemäß sind Blockiermittel vorgesehen, welche das bewegliche Griffteil bei gelöster Betätigungsstange blockieren. Diese Anordnung hat den Vorteil, dass das bewegliche Griffteil bei gelöstem Instrumenteneinsatz in einer definierten Stellung gehalten wird, so dass es leicht und sicher wieder mit der Betätigungsstange verbunden werden kann, wenn der Instrumenteneinsatz an die Instrumentenhandhabe angesetzt wird. Es muss dann nicht mehr, wie bei aus dem Stand der Technik bekannten Instrumenten (siehe z. B. DE 198 09 120 C1), darauf geachtet werden, dass das Griffteil sich in einer bestimmten Position befindet, um mit der Betätigungsstange verbunden werden zu können. Erfindungsgemäß wird das Griffteil automatisch durch die Blockiermittel in derjenigen Position gehalten, in der es sich mit der Betätigungsstange verbinden lässt. Darüber hinaus sind die Blockiermittel so ausgestaltet, dass sie bei richtig verbundener Betätigungsstange das Griffteil wieder freigeben. So ist für den Anwender sichergestellt, dass immer, wenn sich das Griffteil bewegen lässt, der Instrumenteneinsatz mit der Instrumentenhandhabe richtig verbunden ist und die vollständige Funktionsfähigkeit des Instrumentes gegeben ist. Auf diese Weise wird eine sehr einfache Handhabung des erfindungsgemäßen Instrumentes erreicht, Instrumenteneinsatz und Instrumentenhandhabe müssen lediglich zusammengesteckt werden, ohne dass auf eine bestimmte Voreinstellung oder Positionierung einzelner Elemente genauer geachtet werden muss.

Vorzugsweise ist in der Instrumentenhandhabe eine mit dem beweglichen Griffteil verbundene Aufnahme zur Verbindung mit dem proximalen Ende der Betätigungsstange angeordnet, wobei die Blockiermittel ein an der Aufnahme angeordnetes Sperrelement aufweisen, das von einem Federelement in eine blockierende Position und von dem proximalen Ende der Betätigungsstange gegen die Federkraft des Federelementes in eine gelöste Position bewegbar ist. Das Federelement bewirkt somit, dass die Blockiereinrichtung selbstsperrend ausgebildet ist, d. h. bei Entnahme der Betätigungsstange aus der Aufnahme das Griffteil selbsttätig, vorzugsweise in einer vorbestimmten Position, blockiert, so dass das Griffteil bei gelöster Betätigungsstange nicht bewegt werden kann. Dadurch wird auch die Aufnahme für die Betätigungsstange in einer vorbestimmten Position gehalten, so dass die Betätigungsstange leicht wieder mit der Aufnahme verbunden werden kann. Dadurch, dass das Sperrelement von der Betätigungsstange beim Einsetzen wieder in die gelöste Position bewegt wird, werden die Blockiermittel beim Ansetzen des Instrumenteneinsatzes an die Instrumentenhandhabe selbsttätig wieder gelöst, wenn die Betätigungsstange in die Aufnahme eingesetzt wird. Wenn die Betätigungsstange wieder mit der Aufnahme verbunden ist, kann der Anwender somit das Griffteil wieder bewegen, und die ordnungsgemäße Funktion des Instrumentes ist sichergestellt. Die Verbindung zwischen Aufnahme und proximalem Ende der Betätigungsstange ist vorzugsweise so ausgestaltet, dass die Betätigungsstange aus der Aufnahme erst entnommen werden kann, wenn die Aufnahme sich in einer vorbestimmten Position befindet, in welcher dann bei entnommener Betätigungsstange die Aufnahme und das bewegliche Griffteil fixiert werden.

Weiter bevorzugt ist die Rastverbindung zwischen der Betätigungsstange und dem beweglichen Griff teil als Kugelrastverbindung ausgebildet. Eine solche Verbindung ermöglicht ein leichtes Zusammenstecken von Betätigungsstange und Griffteil bzw. von Betätigungsstange und Aufnahme, welche mit dem Griffteil in Verbindung steht.

Dazu ist vorzugsweise am proximalen Ende der Betätigungsstange umfänglich eine Ringnut ausgebildet und die Aufnahme als Zylinderhülse ausgebildet, in welche das proximale Ende der Betätigungsstange einsetzbar ist und in welcher zumindest eine Rastkugel angeordnet ist, welche mit der Ringnut in Eingriff treten kann. Diese Ausgestaltung der Eingriffselemente, d. h. der Ringnut und der Rastkugel, ermöglicht, dass die Rastkugel in jeder beliebigen umfänglichen Position mit der Ringnut in Eingriff treten kann. Dies hat zum einen den Vorteil, dass beim Zusammensetzen des Instrumentes nicht auf eine bestimmte Winkelposition zwischen Instrumenteneinsatz und Instrumentenhandhabe bzw. zwischen Betätigungsstange und Aufnahme geachtet werden muss. Zum anderen bietet diese Ausgestaltung die Möglichkeit, dass der Instrumenteneinsatz gegenüber der Instrumentenhandhabe verdreht werden kann, beispielsweise um bei einer Operation ein am distalen Ende des Instrumenteneinsatzes angeordnetes Zangenmaul in eine gewünschte Winkellage bringen zu können. Die Rastkugel ist zweckmäßigerweise in einer Durchgangsbohrung in der Wandung der Zylinderhülse in einer Richtung quer zur Längsachse der Zylinderhülse beweglich angeordnet, wobei die Rastkugel einen Durchmesser aufweist, welcher größer als die Dicke der Wandung ist. Dies bedeutet, die Rastkugel steht entweder über den Innenumfang der Zylinderhülse oder über den Außenumfang der Zylinderhülse vor. Die Betätigungsstange wird in Längsrichtung der Zylinderhülse in diese eingesetzt, d. h. die Rastkugel ist quer zur Einsetzrichtung der Betätigungsstange bewegbar. Die Betätigungsstange weist an ihrem proximalen Ende einen Außendurchmesser auf, welcher im Wesentlichen dem Innendurchmesser der Zylinderhülse entspricht, so dass die Rastkugel, wenn sie nach innen in die Zylinderhülse hineinragt, in die Ringnut am proximalen Ende der Betätigungsstange eingreifen kann und die Betätigungsstange so in der Zylinderhülse fixieren kann. Um die Betätigungsstange von der Zylinderhülse außer Eingriff zu bringen, muss die Rastkugel radial nach außen bewegt werden, so dass sie am Außenumfang der Zylinderhülse nach außen vorsteht.

Um dies zu ermöglichen, ist an einer Führung, in welcher die Zylinderhülse beweglich geführt ist, eine Ausnehmung oder Schulter vorgesehen, mit welcher die Rastkugel in Eingriff treten kann, um die Rastkugel von der Betätigungsstange außer Eingriff zu bringen und gleichzeitig die Zylinderhülse in der Führung zu blockieren. Die Zylinderhülse ist in der Führung in ihrer Längsrichtung, d. h. auch in Längs- und Bewegungsrichtung der Betätigungsstange, bewegbar, um eine Bewegung des beweglichen Griffteils auf die Betätigungsstange zu übertragen. In einer bestimmten Position in der Führung ist eine Ausnehmung bzw. Schulter vorgesehen, an welcher sich die Führung radial erweitert. An dieser Stelle kann die Rastkugel aus der Durchgangsbohrung radial nach außen austreten, so dass sie im Inneren der Zylinderhülse von der Ringnut am proximalen Ende der Betätigungsstange außer Eingriff treten kann und somit die Betätigungsstange gelöst wird und aus der Zylinderhülse entnommen werden kann. Da die Rastkugel gleichzeitig am Außenumfang der Zylinderhülse in die Ausnehmung in der Führung eintritt bzw. an der Schulter in der Führung anliegt, wird dadurch die Zylinderhülse in der Führung blockiert, d. h. in der gegebenen Position festgehalten, so dass auch das Griffteil in dieser Position blockiert ist. Die Ausnehmung bzw. Schulter in der Führung ist zweckmäßigerweise so positioniert, dass bei der im Betrieb möglichen Bewegung der Zylinderhülse, welche durch die maximale Linearverschiebung der Betätigungsstange beispielsweise zum Öffnen und Schließen eines Zangenmauls am distalen Ende des Instrumenteneinsatzes begrenzt ist, die Rastkugel in der Zylinderhülse die Position der Schulter bzw. Ausnehmung in der Führung nicht erreicht und somit nicht mit der Ausnehmung oder Schulter in Eingriff treten kann. Dadurch wird sichergestellt, dass im Betrieb die Betätigungsstange stets in der Zylinderhülse gesichert bleibt. Zur Entnahme der Betätigungsstange muss der Instrumenteneinsatz zunächst um ein vorbestimmtes Maß relativ zu der Instrumentenhandhabe bewegt werden, vorzugsweise in Längsrichtung des Instrumenteneinsatzes von der Instrumentenhandhabe weg bewegt werden, wobei die Betätigungsstange zunächst gemeinsam mit der Zylinderhülse relativ zu deren Führung soweit bewegt wird, bis die Zylinderhülse in der Führung eine Position erreicht, welche sie im normalen Betrieb nicht erreicht und an welcher die Rastkugel mit der Ausnehmung bzw. Erweiterung in der Führung in Eingriff treten kann. Bei weiterer Bewegung der Betätigungsstange kann die Rastkugel so von der Ringnut außer Eingriff treten,so dass im Inneren der Zylinderhülse die Betätigungsstange wie beschrieben freigegeben wird. Auf diese Weise wird sichergestellt, dass bei der Entnahme der Betätigungsstange das bewegliche Griffteil und die mit diesem verbundene Aufnahme zunächst in eine vorbestimmte Kupplungsposition bewegt wird, an welcher die Betätigungsstange beim Einsetzen des Instrumenteneinsatzes mit den beweglichen Teilen in der Instrumentenhandhabe gekuppelt wird. Dadurch, dass die Rastkugel sowohl zur Sicherung der Betätigungsstange als auch zum Blockieren der beweglichen Teile in der Instrumentenhandhabe vorgesehen ist, wird sichergestellt, dass immer, wenn die Handhabe gelöst ist, die bewegliche Aufnahme in der Instrumentenhandhabe blockiert ist und umgekehrt. Es kann somit sehr leicht eine selbstsichernde Verbindung geschaffen werden, welche Fehlfunktionen sicher ausschließt.

Weiter bevorzugt ist das Sperrelement im Inneren der Zylinderhülse angeordnet und hält in seiner blockierenden Position die Rastkugel mit der Ausnehmung oder Schulter in der Führung in Eingriff und gibt in seiner gelösten Position die Rastkugel frei. Das Sperrelement ist so ausgebildet, dass es dann, wenn die Betätigungsstange aus der Zylinderhülse entnommen ist, die Rastkugel in ihrer radial nach außen ausgerückten Position, in welcher die Rastkugel die Zylinderhülse an deren Führung sichert, hält. Dazu kann das Sperrelement beispielsweise als ein im Inneren der Zylinderhülse beweglicher Kolben ausgebildet sein, welcher durch ein Federelement distalwärts gedrückt wird. Wenn die Betätigungsstange in die Zylinderhülse bzw. Aufnahme eingesetzt wird, drückt die proximale Stirnseite der Betätigungsstange den Kolben proximalwärts, so dass die Betätigungsstange in die Zylinderhülse eingeschoben werden kann, bis die Ringnut an der Betätigungsstange mit der Rastkugel in Eingriff tritt. Wenn die Betätigungsstange aus der Zylinderhülse entnommen wird, drückt das Federelement den Kolben so weit distalwärts, dass er in den Bereich der Zylinderhülse gelangt, in dem die Rastkugel angeordnet ist. Der Kolben liegt dabei im Wesentlichen an der Innenwandung der Zylinderhülse an, so dass die Rastkugel nicht in das Innere der Zylinderhülse eintreten kann, sondern radial nach außen ausgelenkt wird und in der Aufnahme bzw. an der Schulter der Führung der Zylinderhülse gehalten wird.

Gemäß einer weiteren bevorzugten Ausführungsform weist der Instrumenteneinsatz einen Instrumentenschaft auf, dessen proximales Ende als Lueranschluss ausgebildet ist, welcher zum Verbinden mit der Instrumentenhandhabe und zum Anschluss einer Schlauchleitung dient. Diese Ausgestaltung hat den Vorteil, dass das proximale Ende des Instrumentenschaftes zum Spülen leicht mit üblichen Schlauchleitungen verbunden werden kann. Es sind keine zusätzlichen Reinigungsadapter erforderlich, wie es bei bekannten Instrumenten üblicherweise der Fall ist. Somit erfordert das erfindungsgemäße Instrument keine losen Zusatzteile, welche lediglich zur Reinigung benötigt werden und leicht verloren gehen können. Der Lueranschluss dient vorzugsweise gleichzeitig zur Zentrierung des Instrumentenschaftes an der Instrumentenhandhabe. Dazu wird der Lueranschluss in eine zylindrische Ausnehmung an der Instrumentenhandhabe in Längsrichtung des Instrumentenschaftes eingesetzt, wobei der Außenumfang des Lueranschlusses an der Innenwandung der zylindrischen Ausnehmung zur Anlage kommt.

Ferner weist der Instrumenteneinsatz vorzugsweise einen Instrumentenschaft auf, welcher lösbar mit der Instrumentenhandhabe über eine Rastverbindung verbindbar ist. Dies ermöglicht, dass der gesamte Instrumenteneinsatz leicht durch Verrasten mit der Instrumentenhandhabe verbindbar ist und ebenfalls leicht wieder von der Instrumentenhandhabe gelöst werden kann. So ist eine problemlose Austauschbarkeit des Instrumentenschaftes gewährleistet. Vorzugsweise ist die Schnittstelle zwischen Instrumentenschaft und Instrumentenhandhabe so ausgebildet, dass unterschiedliche Instrumenteneinsätze mit ein und derselben Instrumentenhandhabe verbunden werden können.

Gemäß einer ersten bevorzugten Ausführungsform weist die Rastverbindung zwischen Instrumentenschaft und Instrumentenhandhabe einen an der Instrumentenhandhabe angeordneten, quer zur Längsachse des Instrumentenschaftes bewegbaren Rastbolzen auf, welcher mit einer Ausnehmung am proximalen Ende des Instrumentenschaftes lösbar in Eingriff treten kann. Der Rastbolzen ist dazu vorzugsweise durch ein Federelement derart vorgespannt, dass er selbsttätig in die Ausnehmung am proximalen Ende des Instrumentenschaftes eintritt, um den Instrumentenschaft an der Instrumentenhandhabe zu sichern. Zum Lösen ist an dem Rastbolzen eine Druckfläche vorgesehen, über welche der Anwender den Rastbolzen in eine gelöste Position gegen die Federkraft des Federelementes drücken kann, um den Rastbolzen von dem Instrumentenschaft außer Eingriff zu bringen, so dass dieser von der Instrumentenhandhabe abgenommen werden kann.

Vorzugsweise hintergreift der Rastbolzen dabei die Auskragungen des Lueranschlusses. Somit sind keine zusätzlichen Eingriffselemente am proximalen Ende des Zangenschaftes erforderlich. Darüber hinaus sind die Auskragungen des Lueranschlusses als umfänglicher, ringförmiger Vorsprung ausgebildet, so dass der Rastbolzen in jeder Winkellage des Instrumentenschaftes bezüglich seiner Längsachse die Auskragung des Lueranschlusses hintergreifen kann. Es muss somit zum einen beim Einsetzen des Instrumentenschaftes nicht auf eine bestimmte Winkelstellung geachtet werden, so dass das Einsetzen vereinfacht wird. Zum anderen kann das Instrument so ausgestaltet werden, dass der Instrumentenschaft und damit der Instrumenteneinsatz auch im Betrieb relativ zu der Instrumentenhandhabe um die Längsachse des Instrumentenschaftes drehbar ist, beispielsweise um ein Zangenmaul am distalen Ende des Instrumenteneinsatzes in eine gewünschte Position bringen zu können. Da die Ausnehmung am proximalen Ende des Instrumentenschaftes, vorzugsweise durch den Lueranschluss gebildet, rotationssymmetrisch ist, muss dazu die Rastverbindung zwischen Instrumentenschaft und Instrumentenhandhabe nicht gelöst werden.

Gemäß einer weiteren bevorzugten Ausführungsform ist die Rastverbindung zwischen Instrumentenschaft und Instrumentenhandhabe als Kugelrastverbindung ausgebildet. Die Kugelrastverbindung ermöglicht einen besonders kompakten Aufbau der Rastverbindung und gleichzeitig eine leichtgängige und sichere Verbindung zwischen Instrumentenschaft und Instrumentenhandhabe.

Die Kugelrastverbindung zwischen Instrumentenschaft und Instrumentenhandhabe weist vorzugsweise ein Verschlusselement zur Sicherung der Kugelrastverbindung und ein Griffelement auf, wobei das Verschlusselement sowohl durch Bewegung des Griffelementes als auch unabhängig von der Bewegung des Griffelementes in eine entsicherte Position bewegbar ist. Dies ermöglicht, dass zum Lösen des Instrumentenschaftes von der Instrumentenhandhabe das Griffelemente bewegt werden kann, um das Verschlusselement in eine entsicherte Position zu bringen, in welcher der Instrumentenschaft von der Instrumentenhandhabe abgenommen werden kann. Dadurch, dass das Verschlusselement zusätzlich unabhängig von dem Griffelement bewegbar ist, ist es beim Zusammensetzen von Instrumentenschaft und Instrumentenhandhabe jedoch nicht notwendig, das Griffelement zu bewegen. Vielmehr verbleibt das Griffelement beim Zusammensetzen des Instrumentes in einer bestimmten Position, so dass ein Benutzer den jeweiligen Instrumententeil am Griffelement festhalten kann und mit dem anderen Instrumententeil zusammenstecken kann, je nachdem, ob Griff- und Verschlusselement an einer Instrumentenhandhabe oder am Instrumentenschaft angebracht sind. Beim Zusammensetzen von Instrumentenschaft und Instrumentenhandhabe wird das Verschlusselement lediglich über das zu verbindende Gegenstück bewegt und muss nicht manuell durch Bewegung des Griffteiles in die entsicherte Position bewegt werden. Das heißt, wenn das Verschlusselement an der Instrumentenhandhabe angeordnet ist, wird das Verschlusselement durch Einsetzen des Instrumentenschaftes in seine entsicherte Position bewegt. Umgekehrt, für den Fall, dass das Verschlusselement am Instrumentenschaft angeordnet ist, kann das Verschlusselement durch ein korrespondierendes Teil an der Instrumentenhandhabe in seine entsicherte Position bewegt werden, wenn beide Teile zusammengefügt werden.

Weiter bevorzugt weist die Kugelrastverbindung zwischen Instrumentenschaft und Instrumentenhandhabe eine Verschlusshülse zur Sicherung der Kugelrastverbindung und eine Griffhülse auf, wobei die Verschlusshülse in einer gesicherten Position die Bewegung zumindest einer Rastkugel blockiert und in einer entsicherten Position die Rastkugel freigibt und die Verschlusshülse sowohl durch Bewegung der Griffhülse als auch unabhängig von einer Bewegung der Griffhülse in die entsicherte Position bewegbar ist. Rastkugeln sind beispielsweise an dem Instrumentenschaft vorgesehen und können mit entsprechenden Ausnehmungen, vorzugsweise einer Ringnut, an einem Element der Instrumentenhandhabe zur Sicherung des Instrumentenschaftes an der Instrumentenhandhabe in Eingriff treten. Wenn die Rastkugeln mit der Ringnut in Eingriff sind, werden sie durch die Verschlusshülse in dieser Position gesichert, so dass sie aus den Ausnehmungen bzw. der Nut nicht außer Eingriff treten können. Zum Lösen muss die Verschlusshülse von den Rastkugeln weg bewegt werden, so dass diese aus der Ausnehmung bzw. Nut austreten und das entsprechende Element freigeben können. Dazu kann die Verschlusshülse über die Griffhülse in eine gelöste bzw. entsicherte Position bewegt werden. Auch um die Rastkugeln wieder mit der Nut in Eingriff zu bringen, wenn der Instrumentenschaft an die Instrumentenhandhabe angesetzt wird, muss zunächst die Verschlusshülse in die entsicherte Position bewegt werden, damit die Kugeln mit der Nut in Eingriff treten können. Hierzu ist die Verschlusshülse so ausgestaltet, dass sie sich unabhängig von der Griffhülse in die entsicherte Position bewegen kann, wobei die Griffhülse in ihrer ursprünglichen Lage verbleibt. Dies ermöglicht, dass das Instrument beim Zusammensetzen an der Griffhülse festgehalten werden kann, ohne dass die Griffhülse sich bewegen muss. Dadurch wird das Zusammensetzen des Instruments erheblich vereinfacht.

Vorzugsweise ist die Verschlusshülse im Inneren der Griffhülse angeordnet und wird durch ein Federelement in der gesicherten Position gehalten, wobei die Verschlusshülse zumindest eine Anlageschulter aufweist, welche von einer Anlageschulter der Griffhülse nur in einer der Federkraft des Federelementes entgegengesetzten Richtung hintergriffen wird. Die Anlageschulter der Verschlusshülse ist vorzugsweise an deren Außenumfang und die Anlageschulter der Griffhülse an deren Innenumfang ausgebildet. Die Hintergreifung der Verschlusshülse durch die Griffhülse nur in der genannten Richtung bewirkt, dass die Verschlusshülse durch Bewegung der Griffhülse entgegen der Federkraft des Federelementes bewegt werden kann. Andererseits kann sich die Verschlusshülse auch unabhängig von der Griffhülse entgegen der Federkraft des Federelementes bewegen, wobei die Anlageschultern von Griffhülse und Verschlusshülse außer Eingriff treten. Wird nur die Verschlusshülse bewegt, wird somit das Griffelement nicht mitbewegt. Darüber hinaus wird über das Federelement und die Verschlusshülse sowie die Anlageschultern auf die Griffhülse eine Kraft aufgebracht, welche dafür sorgt, dass die Griffhülse durch das Federelement ebenfalls in eine vorbestimmte Position gedrückt wird und lediglich zum Lösen der Kugelrastverbindung entgegen der Federkraft gemeinsam mit der Verschlusshülse bewegt werden muss. Beim Zusammensetzen von Instrumentenschaft und Instrumentenhandhabe wird durch ein einzusetzendes Bauteil lediglich die Verschlusshülse gegen die Federkraft bewegt, während die Griffhülse in ihrer ursprünglichen Lage verbleibt.

Die Verschlusshülse und die Griffhülse sind vorzugsweise an der Zangenhandhabe beweglich geführt, und die Griffhülse kommt, wenn die Verschlusshülse in ihrer gesicherten Position ist, an einem Anschlag der Instrumentenhandhabe zur Anlage. Das bedeutet, das Federelement, welches die Verschlusshülse in ihre gesicherte Position drückt, drückt über die Anlageschultern zwischen Verschlusshülse und Griffhülse die Griffhülse gegen den Anschlag an der Instrumentenhandhabe, d. h. die Griffhülse wird ebenfalls über das Federelement in einer vorbestimmten Ruhelage gehalten. Ferner ermöglicht die Griffhülse, wenn sie ergriffen wird, die Instrumentenhandhabe leicht mit dem Instrumentenschaft zusammenzustecken, da die Griffhülse, wenn sie in der Verbindungsrichtung, d. h. distalwärts, bewegt wird, an dem Anschlag zur Anlage kommt und somit die gesamte Instrumentenhandhabe mitbewegt. Wenn die Griffhülse und das Verschlusselement an der Instrumentenhandhabe vorgesehen sind, ist das Federelement so angeordnet, dass es die Verschlusshülse distalwärts in eine gesicherte Position drückt. Umgekehrt ist es auch möglich, Verschluss- und Griffhülse am proximalen Ende des Instrumentenschaftes anzuordnen. In diesem Fall drückt das Federelement das Verschlusselement proximalwärts. Auch der Anschlag zwischen Griffelement und Zangenschaft ist dann so gewählt, dass durch Bewegung der Griffhülse über den Anschlag der Instrumentenschaft proximalwärts auf die Instrumentenhandhabe zu bewegt werden kann.

Nachfolgend wird die Erfindung beispielhaft anhand der beigefügten Figuren beschrieben. In diesen zeigt:
- Fig. 1: eine Schnittansicht des proximalen Endes eines Instrumenteneinsatzes und des distalen Endes einer Instrumentenhandhabe gemäß einer ersten Ausführungsform im gelösten Zustand,
- Fig. 2: eine Schnittansicht der Ausführungsform gemäß Fig. 1 im zusammengesetzten Zustand,
- Fig. 3: eine Schnittansicht des proximalen Endes eines Instrumenteneinsatzes und des distalen Endes einer Instrumentenhandhabe gemäß einer zweiten Ausführungsform im gelösten Zustand,
- Fig. 4: eine Schnittansicht einer Instrumentenhandhabe mit angesetztem Instrumentenschaft gemäß einer dritten Ausführungsform der Erfindung und
- Fig. 5: eine Schnittansicht einer Instrumentenhandhabe mit angesetztem Instrumenteneinsatz gemäß einer vierten Ausführungsform der Erfindung.

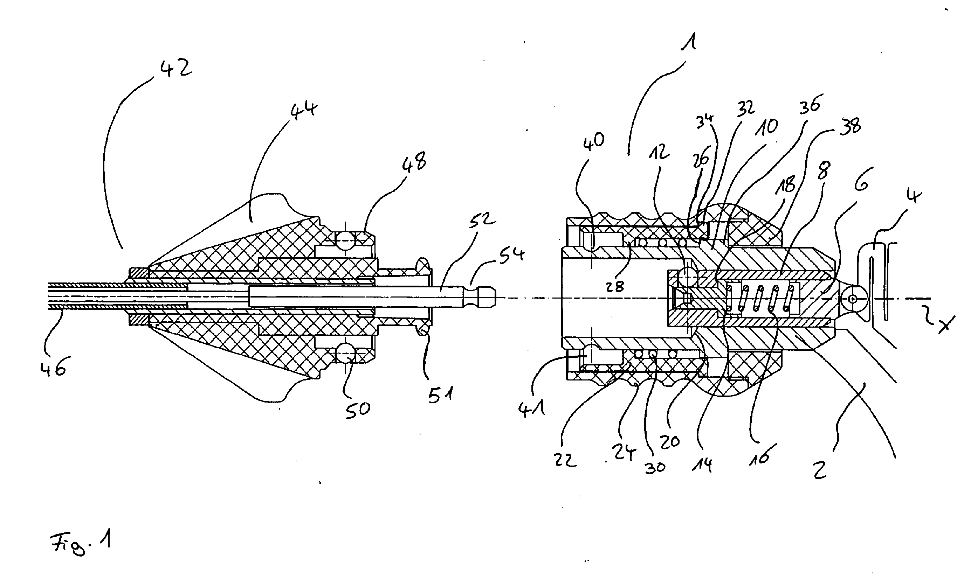

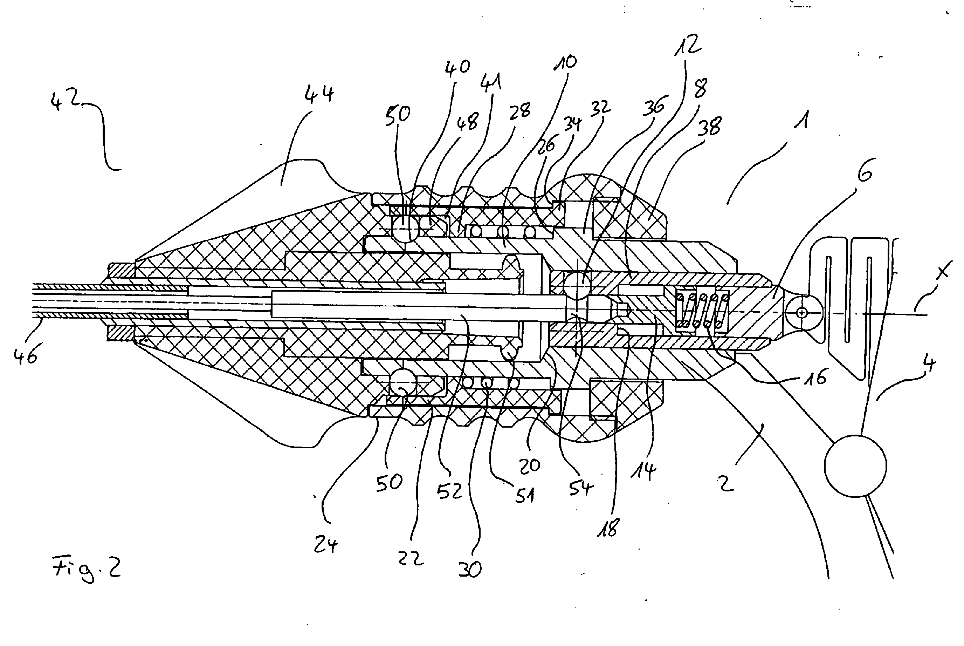

Anhand von Figuren 1 und 2 wird eine erste bevorzugte Ausführungsform der Erfindung erläutert. Fig. 1 zeigt das distale Ende einer Instrumentenhandhabe, beispielsweise einer Zangenhandhabe, sowie das proximale Ende eines Instrumenteneinsatzes, beispielsweise eines Zangeneinsatzes, im Schnitt. Die Instrumentenhandhabe weist ein festes Griffteil 2 sowie ein bewegliches Griffteil 4 auf, welches über ein Verbindungsteil 6 mit einer Zylinderhülse 8 verbunden ist. Das Verbindungsteil 6 ist fest mit der Zylinderhülse 8 verbunden. Die Zylinderhülse 8 ist in einer Zylinderaufnahme 10, welche mit dem festen Griffteil 2 fest verbunden ist, in Richtung der Instrumentenlängsachse X beweglich geführt. Im Bereich des distalen Endes der Zylinderhülse 8 sind radial nach außen gerichtete Durchgangsbohrungen angeordnet, in welchen Rastkugeln 12 angeordnet sind. Im vorliegenden Fall sind drei gleichmäßig über den Umfang verteilte Durchgangsbohrungen mit jeweils einer Rastkugel 12 vorgesehen. Die Rastkugeln 12 weisen einen Durchmesser auf, welcher größer als die Wandstärke der Zylinderhülse 8 ist, so dass die Rastkugeln jeweils entweder nach außen oder nach innen an der Zylinderhülse auskragen.

Im Inneren der Zylinderhülse ist ein Sperrelement 14 in Form eines Kolbens in Richtung der Instrumentenlängsachse X beweglich geführt. Das Sperrelement 14 wird von einer Feder 16 distalwärts gegen einen Absatz 18 im Inneren der Zylinderhülse 8 gedrückt. Das distale Ende des Sperrelementes 14 ragt in dieser Position in den distalen Aufnahmebereich der Zylinderhülse 8 soweit hinein, dass es in Richtung der Längsachse X die Position der Rastkugeln 12 überdeckt. Dabei liegt das Sperrelement 14 am Innenumfang der Zylinderhülse 8 derart an, dass die Rastkugeln 12 nach außen gedrückt werden und am Außenumfang der Zylinderhülse 8 nach außen vorkragen. Dabei kommen die Rastkugeln 12 an einer Anlageschulter 20 im Inneren der Zylinderaufnahme 10 zur Anlage. Die Rastkugeln 12 liegen dabei distalseitig bezüglich der Anlageschulter 20, so dass die Zylinderhülse 8 nicht in Richtung der Längsachse X proximalwärts im Inneren der Zylinderaufnahme 10 bewegt werden kann. Die Rastkugeln 12 halten die Zylinderhülse 8 somit fest in der in Fig. 1 gezeigten Position, in welcher die Zylinderhülse 8 und damit das bewegliche Griffteil 4 gegen eine Bewegung in den gelösten Zustand blockiert sind.

Am Außenumfang der Zylinderaufnahme 10 sind ferner eine Verschlusshülse 22 sowie eine Griffhülse 24 angeordnet, wobei die Verschlusshülse 22 im Inneren der Griffhülse 24 zwischen der Griffhülse 24 und dem Außenumfang der Zylinderaufnahme 10 angeordnet ist. Die Verschlusshülse 22 ist in Richtung der Längsachse X beweglich auf dem Außenumfang der Zylinderaufnahme 10 geführt. Dabei weist die Zylinderaufnahme 10 an ihrem Außenumfang eine distalwärts gerichtete Schulter 26 und die Verschlusshülse einen radial nach innen gerichteten Vorsprung 28 auf, welcher am Außenumfang der Zylinderaufnahme 10 anliegt. Zwischen der Schulter 26 und dem Vorsprung 28 ist eine Druckfeder 30 in Form einer Schraubenfeder angeordnet, welche die Verschlusshülse 22 in die gezeigte, gesicherte Position drückt.

Die Griffhülse 24 ist ebenfalls in Richtung der Längsachse X beweglich am Außenumfang der Zylinderaufnahme 10 geführt. Die Verschlusshülse 22 weist an ihrem proximalen Ende einen nach außen gerichteten, ringförmigen Vorsprung 32 auf, welcher mit seiner distalwärts gerichteten Seite an einer proximalwärts gerichteten Schulter 34 im Inneren der Griffhülse 24 anliegt. In proximaler Richtung tritt der Vorsprung 32 nicht mit der Griffhülse 24 in Eingriff, sondern ist frei in der Griffhülse beweglich. Dies bewirkt, dass die Griffhülse über den Vorsprung 32 und die Schulter 34 durch die Druckfeder 30 ebenfalls distalwärts gedrückt wird, bei Bewegung der Verschlusshülse 22 in proximaler Richtung aber nicht mitbewegt wird. Dabei wird ein Anschlag durch einen ringförmigen Vorsprung 36 am Außenumfang der Zylinderaufnahme 10 gebildet, welcher vom proximalen Ende 38 der Griffhülse 24 hintergriffen wird. Im Bereich des distalen Endes der Zylinderaufnahme 10 ist an deren Außenumfang eine Ringnut 40 ausgebildet, welche von der Verschlusshülse 22 in deren gesicherter Position überdeckt wird, wobei die Verschlusshülse 22 in radialer Richtung von der Ringnut 40 beabstandet ist, so dass ein ringförmiger Freiraum 41 gebildet wird

Der Instrumenteneinsatz 42 weist an seinem proximalen Ende einen Drehgriff 44 auf, welcher fest mit dem proximalen Ende eines Instrumentenschaftes 46 verbunden ist, um diesen relativ zur Instrumentenhandhabe um die Längsachse X drehen zu können. Im Bereich des proximalen Endes weist der Drehgriff 44 einen zylindrischen Eingriffsabschnitt 48 auf, welcher in den Freiraum 41 zwischen Verschlusshülse 22 und Zylinderaufnahme 10 der Instrumentenhandhabe von deren distaler Seite her eintreten kann. In dem Eingriffsabschnitt 48 sind radialwärts gerichtete Durchgangsbohrungen vorgesehen, in welchen Rastkugeln 50 angeordnet sind. Im gezeigten Beispiel sind drei gleichmäßig über den Umfang des Eingriffsabschnittes 48 verteilte Durchgangsbohrungen mit Rastkugeln 50 vorgesehen. Die Rastkugeln 50 können mit der Ringnut 40 in Eingriff treten, um den Instrumenteneinsatz 42 an der Instrumentenhandhabe 1 zu sichern. Das proximale Ende des Zangenschaftes 46, welches im Inneren des Eingriffsabschnittes 48 angeordnet ist und über diesen proximalwärts vorsteht, ist als Lueranschluss 51 ausgebildet, welcher in das Innere der Zylinderaufnahme 10 eintreten kann und an deren Innenumfang zur Anlage kommt. Dadurch wird der Instrumenteneinsatz 42 bezüglich der Aufnahme in der Instrumentenhandhabe 1 zentriert. An den Lueranschluss 51 kann sehr einfach eine Spülleitung zum Durchspülen des Instrumentenschaftes 46 angeschlossen werden, wenn der Instrumenteneinsatz 21 von der Instrumentenhandhabe getrennt ist.

Im Inneren des Instrumentenschaftes 46 ist eine Betätigungsstange 52 angeordnet, welche sich in Richtung der Längsachse X zum distalen Ende des Instrumenteneinsatzes 42 erstreckt, um dort beispielsweise ein nicht gezeigtes Zangenmaul zu bewegen. Das proximale Ende der Betätigungsstange 52 ragt proximalseitig aus dem Lueranschluss 50 heraus und weist eine umfängliche Ringnut 54 auf. Das proximale Ende der Betätigungsstange kann ferner profiliert sein, z.B. plane Seitenflächen bei sonst rundem Querschnitt aufweisen, um eine bessere Durchspülbarkeit des Instrumentenschaftes zu ermöglichen. Die Ringnut 54 kann mit den Rastkugeln 12 in der Zylinderhülse 8 zur Verriegelung der Zylinderhülse 8 an der Betätigungsstange 52 in Eingriff treten.

Fig. 2 zeigt eine Schnittansicht der Anordnung gemäß Fig. 1 im verbundenen Zustand. Zum Verbinden des Instrumenteneinsatzes 42 mit der Instrumentenhandhabe 1 wird diese an der Griffhülse 24 und/oder den Griffteilen 2 und 4 ergriffen, während der Instrumenteneinsatz 42 vorzugsweise an dem Drehgriff 44 ergriffen wird. Dann werden Instrumenteneinsatz 42 und Instrumentenhandhabe 1 aufeinander zu bewegt. Wenn die Griffhülse 24 ergriffen wird und in Richtung der Längsachse X auf den Instrumenteneinsatz 42 zu bewegt wird, wird über den Vorsprung 36 ebenfalls die Zylinderaufnahme 10 und somit die gesamte Instrumentenhandhabe 1 auf den Instrumenteneinsatz 42 zubewegt. Beim Zusammenfügen tritt der Lueranschluss 50 in das Innere der Zylinderaufnahme 10 ein und, wenn Instrumenteneinsatz 42 und Instrumentenaufnahme 1 weiter zusammenbewegt werden, tritt das proximale Ende der Betätigungsstange 52 mit der Ringnut 54 in das Innere der Zylinderhülse 8 ein. Dabei wird das Sperrelement 14 gegen die Federkraft der Feder 16 proximalwärts bewegt, bis die Ringnut 54 eine Position erreicht, in welcher die Rastkugeln 12 in die Ringnut 54 eintreten können.

Dadurch wird die Betätigungsstange 52 formschlüssig mit der Zylinderhülse 8 verbunden. Gleichzeitig treten die Rastkugeln 12 von der Anlageschulter 20 außer Eingriff, so dass bei weiterer proximalwärtiger Bewegung des Instrumenteneinsatzes 42 die gesamte Zylinderhülse 8 proximalwärts bewegt wird. Dabei tritt die Zylinderhülse 8 so weit in die proximalseitige Aufnahme der Zylinderaufnahme 10 ein, dass die radialwärts gerichteten Durchgangsbohrungen mit den Rastkugeln 12 von der Innenwandung der Zylinderaufnahme 10 direkt überdeckt werden, so dass die Rastkugeln 12 in Eingriff mit der Ringnut 54 gehalten werden, so dass die Betätigungsstange 52 nicht mehr von der Zylinderhülse 8, welche mit dem beweglichen Griffteil 4 verbunden ist, außer Eingriff treten kann.

Gleichzeitig tritt beim Verbinden der Instrumentenhandhabe 1 mit dem Instrumenteneinsatz 42 der Eingriffsabschnitt 48 in den Raum 41 zwischen der Zylinderaufnahme und der Verschlusshülse 22 ein. Dabei stoßen die Rastkugeln 50 zunächst an der distalseitigen Stirnkante der Verschlusshülse 22 an, welche dann gegen die Federkraft der Druckfeder 30 soweit proximalwärts bewegt wird, dass die Rastkugeln 50 über der Ringnut 40 zu liegen kommen und in diese eintreten können. Da die Verschlusshülse 22 in proximaler Richtung nicht an der Griffhülse 24 zur Anlage kommt, wird bei dieser Bewegung der Verschlusshülse 22 die Griffhülse 24 nicht mitbewegt, so dass die Griffhülse 24 in ihrer ursprünglichen Lage verbleibt und weiter festgehalten werden kann, um Instrumentenhandhabe und Instrumenteneinsatz 42 zusammenzuführen.

Wenn die Rastkugeln 50 in die Ringnut 40 eintreten, bewegen sich die Rastkugeln 50 in ihren Durchgangsbohrungen in dem Eingriffsabschnitt 48 radial nach innen, so dass sie mit der Stirnseite der Verschlusshülse 22 außer Eingriff treten und die Verschlusshülse 22 durch die Druckfeder 30 wieder distalwärts bewegt wird, bis der distale Bereich der Verschlusshülse 22 die Durchgangsbohrungen, in welchen die Rastkugeln 50 angeordnet sind, überdeckt. In dieser Position wird durch die Verschlusshülse 22 verhindert, dass sich die Rastkugeln 50 radialwärts nach außen bewegen und von der Ringnut 40 außer Eingriff treten können. In dieser Lage wird der Instrumentenschaft 46 somit durch die Verschlusshülse 22 an der Instrumentenhandhabe 1 gesichert.

Da die Eingriffselemente in Form von Ringnuten 54 und 40 und Rastkugeln 12 und 50 ausgebildet sind, lässt sich im zusammengefügten Zustand der Instrumenteneinsatz 42 durch Drehung an dem Drehgriff 44 relativ zu der Instrumentenhandhabe 1 um die Längsachse X drehen. Durch eine Riffelung der Ringnut 40 in Umfangsrichtung kann ferner eine feinrastende Drehbarkeit ermöglicht werden. Darüber hinaus muss beim Zusammenfügen aufgrund der rotationssymmetrischen Ausgestaltung der ineinander greifenden Teile nicht auf eine bestimmte Winkelposition des Instrumenteneinsatzes 42 relativ zu der Instrumentenhandhabe 1 geachtet werden.

Zum Lösen der Verbindung zwischen Instrumenteneinsatz 42 und Instrumentenhandhabe 1 wird die Griffhülse 24 in Richtung der Längsachse X proximalwärts bewegt, wobei die Griffhülse 24 über die Schulter 26 mit dem Vorsprung 32 der Verschlusshülse 22 in Eingriff kommt und die Verschlusshülse 22 in proximaler Richtung mitbewegt. Wenn die Verschlusshülse 22 durch Bewegung der Griffhülse 24 so weit proximalwärts bewegt worden ist, dass die Verschlusshülse 22 die Rastkugeln 50 nicht mehr überdeckt, können die Rastkugeln 50, wenn der Instrumenteneinsatz 42 relativ zu der Instrumentenhandhabe 1 distalwärts bewegt wird, sich radial nach außen bewegen und von der Ringnut 40 außer Eingriff treten. Wenn Instrumentenhandhabe 1 und Instrumenteneinsatz 42 zueinander bewegt werden, bleibt die Betätigungsstange 52 zunächst mit der Zylinderhülse 8 in Eingriff, so dass die Zylinderhülse 8 relativ zu der Zylinderaufnahme 10 distalwärts bewegt wird, bis die Rastkugeln 12 aus der proximalseitigen Aufnahme der Zylinderaufnahme austreten und eine Position distal von der Anlageschulter 20 erreichen. In dieser Position können sich die Rastkugeln 12 radial nach außen bewegen und von der Ringnut 54 außer Eingriff treten, so dass die Betätigungsstange 52 aus der Zylinderhülse 8 distalwärts herausgezogen werden kann. Gleichzeitig wird das Sperrelement 14 durch die Feder 16 distalwärts gedrückt, wobei es mit seiner Stirnseite an der proximalen Stirnseite der Betätigungsstange 52 anliegt. Dabei bewegt sich das Sperrelement 14 so weit distalwärts, bis es die Durchgangsbohrungen mit den Rastkugeln 12 überdeckt und somit die Rastkugeln 12 in ihrer radial ausgelenkten Position, in welcher sie die Anlageschulter 20 hintergreifen, sichert. Das Sperrelement 14 kommt dann an dem Absatz 18 zur Anlage. Wenn die Rastkugeln 12 die Anlageschulter 20 hintergreifen, ist die Zylinderhülse 8 in der Zylinderaufnahme 10 blockiert, so dass der bewegliche Griffteil 4 nicht mehr bewegt werden kann. Auf diese Weise wird zum einen sichergestellt, dass der bewegliche Griffteil 4 nur bewegt werden kann, wenn der Instrumenteneinsatz 42 ordnungsgemäß mit der Instrumentenhandhabe 1 verbunden ist. Ferner wird die Verbindung von Instrumenteneinsatz 42 und Instrumentenhandhabe 1 erleichtert, da die Zylinderhülse 8, welche als Aufnahme für die Betätigungsstange 52 dient, durch die Blockierung durch die Rastkugeln 12 in einer definierten Position gehalten wird.

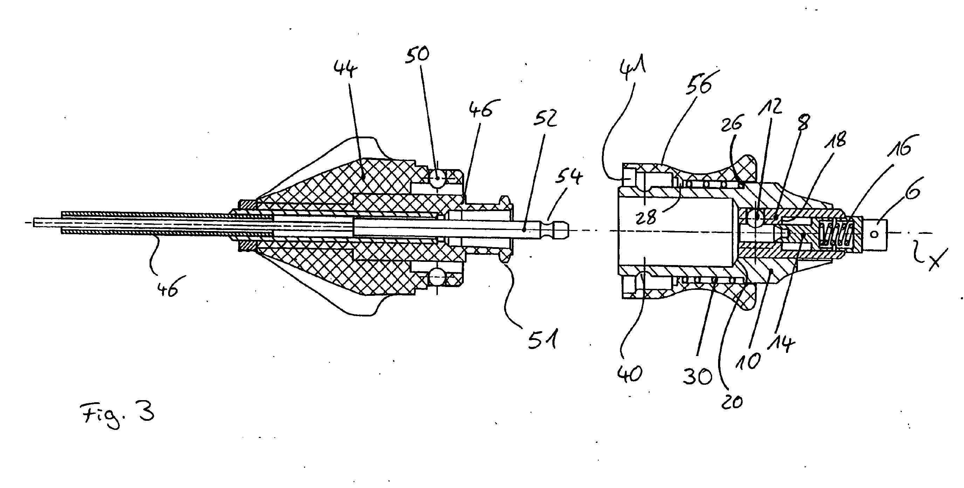

Fig. 3 zeigt eine Ausführungsform ähnlich zu der in Fig. 1 gezeigten Ausführungsform, wobei hier die Verschlusshülse 22 und das Griffteil 24 als einstückiges Griffteil 56 ausgebildet sind. Alle übrigen Elemente entsprechen denen anhand von Figuren 1 und 2 beschriebenen Elementen, so dass auch die Funktionsweise identisch ist. Der einzige Unterschied in der Funktionsweise der Anordnung gemäß Fig. 3 zu den vorangehend beschriebenen Anordnungen liegt darin, dass bei der Ausführungsform gemäß Fig. 3 die Griffhülse 56, welche die Elemente der Griffhülse 24 und der Verschlusshülse 22 gemäß den Ausführungsformen in Figuren 1 und 2 vereinigt, ebenfalls in Richtung der Längsachse X proximalwärts bewegt werden muss, um die Instrumentenhandhabe 1 mit dem Instrumenteneinsatz 42 zu verbinden.

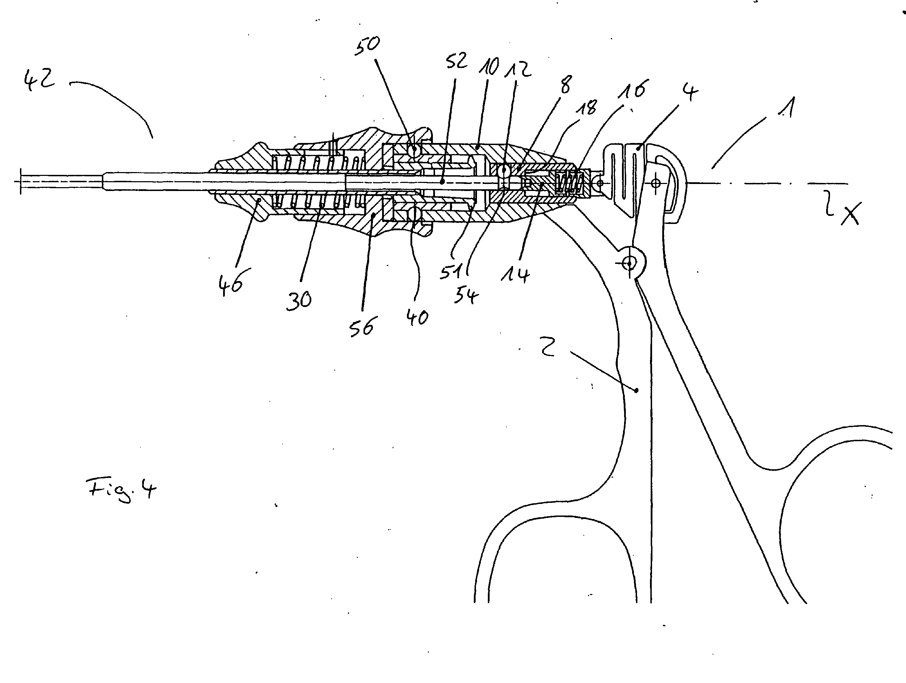

Fig. 4 zeigt in einer Schnittansicht eine weitere Ausführungsform der Erfindung. Auch die Ausführungsform gemäß Fig. 4 entspricht im Wesentlichen den vorangehend beschriebenen Ausführungsformen und insbesondere der anhand von Fig. 3 beschriebenen Ausführungsform. Der Unterschied der dritten Ausführungsform gemäß Fig. 4 zu den vorangehend beschriebenen Ausführungsformen liegt darin, dass die Rastkugeln 50 bei dieser Ausführungsform auf Seiten der Instrumentenhandhabe und die Ringnut 40 sowie die Griffhülse 56 auf Seiten des Instrumenteneinsatzes angeordnet sind. Die Funktionsweise entspricht jedoch der vorangehenden Beschreibung. Die Blockierung der Griffteile bei entnommener Betätigungsstange 52 entspricht den vorangehend beschriebenen Ausführungsformen. Auch bei der Ausführungsform mit Verschlusshülse 22 ist eine solch umgekehrte Anordnung der Kupplungselemente an Instrumentenschaft und -handhabe möglich.

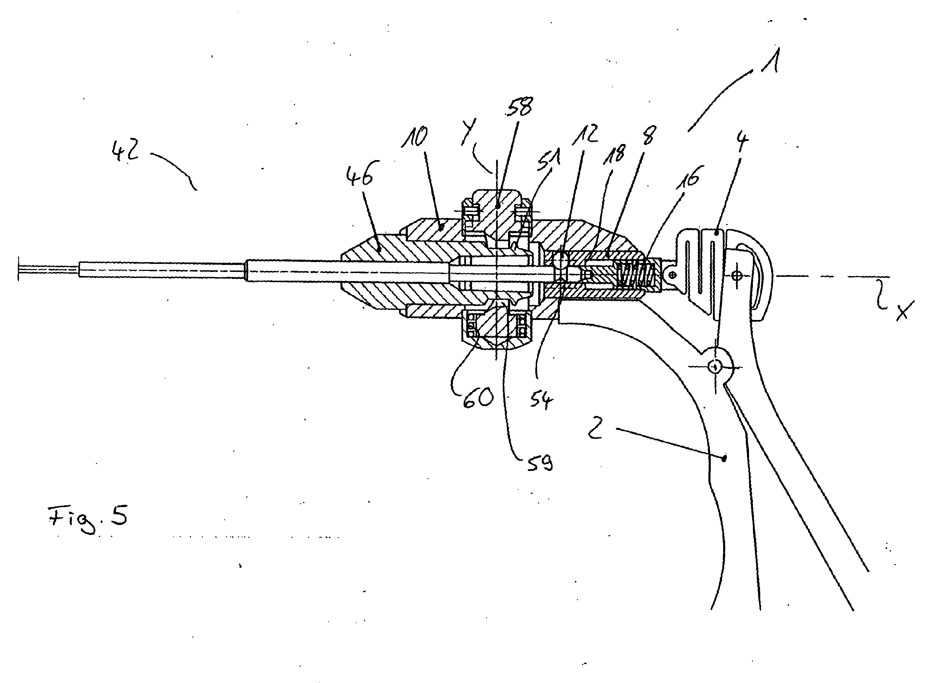

Fig. 5 zeigt eine weitere Ausführungsform, welche sich wiederum lediglich in der Art der Verbindung zwischen Instrumentenschaft 46 und der Instrumentenhandhabe 1 von den vorangehend beschriebenen Ausführungsformen unterscheidet. Die Funktionsweise der Blockiermittel zum Blockieren der Handhabe bei entnommener Betätigungsstange 52 entspricht der anhand von Figuren 1 und 2 im Detail beschriebenen Ausgestaltung. Die Verbindung zwischen Instrumentenhandhabe 1 und Instrumenteneinsatz 42 bzw. dem proximalen Ende des Instrumentenschaftes 46 erfolgt gemäß der Ausführungsform in Fig. 5 über einen Rastbolzen 58. Der Rastbolzen 58 ist in der Zylinderaufnahme 10 angeordnet und erstreckt sich quer zur Längsachse X durch die Zylinderaufnahme 10 hindurch, wobei er in einer Richtung der Achse Y normal zur Längsachse X beweglich ist. In Richtung der Längsachse X erstreckt sich quer zu der Achse Y durch den Rastbolzen ein Durchgangsloch 59. Der Rastbolzen 58 wird durch eine Feder 60 in Richtung der Achse Y in eine geschlossene Position gedrückt, in welcher die Umfangskanten des Durchgangsloches 59 den radial nach außen auskragenden Vorsprung des Lueranschlusses 51 zumindest teilweise hintergreifen. Auf diese Weise wird der Instrumentenschaft 46 an der Instrumentenhandhabe 1 gesichert, so dass er nicht in distaler Richtung von der Instrumentenhandhabe 1 abgenommen werden kann. Zum Lösen wird der Rastbolzen 58 manuell gegen die Federkraft der Feder 60 gedrückt, so dass die Umfangskante des Durchgangsloches 59 von der Auskragung des Lueranschlusses 51 außer Eingriff kommt und der Instrumentenschaft 46 von der Zylinderaufnahme 10 bzw. der Instrumentenhandhabe 1 getrennt werden kann. Lösen und Verbinden der Betätigungsstange 52 erfolgt auch bei dieser Ausführungsform, wie anhand von Figuren 1 und 2 beschrieben.

### Bezugszeichenliste

- 1 -: Instrumentenhandhabe
- 2 -: festes Griffteil
- 4 -: bewegliches Griffteil
- 6 -: Verbindungsteil
- 8 -: Zylinderhülse
- 10 -: Zylinderaufnahme
- 12 -: Rastkugeln
- 14 -: Sperrelement
- 16 -: Feder
- 18 -: Absatz
- 20 -: Anlageschulter
- 22 -: Verschlusshülse
- 24 -: Griffhülse
- 26 -: Schulter
- 28 -: Vorsprung
- 30 -: Druckfeder
- 32 -: Vorsprung
- 34 -: Schulter
- 36 -: Vorsprung
- 38 -: proximales Ende der Griffhülse
- 40 -: Ringnut
- 41 -: Freiraum
- 42 -: Instrumenteneinsatz
- 44 -: Drehgriff
- 46 -: Instrumentenschaft
- 48 -: Eingriffsabschnitt
- 50 -: Rastkugeln
- 51 -: Lueranschluss
- 52 -: Betätigungsstange
- 54 -: Ringnut
- 56 -: Griffhülse
- 58 -: Rastbolzen
- 59 -: Durchgangsloch
- 60 -: Feder

- X -: Instrumentenlängsachse
- Y -: Achse

## Patentansprüche

1. Medizinisches Instrument mit einem Instrumenteneinsatz (42) und einer Instrumentenhandhabe (1), welche lösbar miteinander verbindbar sind, wobei eine Betätigungsstange (52) des Instrumenteneinsatzes (42) über eine Rastverbindung (12, 54) lösbar mit einem beweglichen Griffteil (4) der tnstrumentenhandhabe (1) verbindbar ist und das medizinische Instrument Blockiermittel (14) aufweist, welche das bewegliche Griffteil (4) bei gelöster Betätigungsstange (52) blockieren.

2. Medizinisches Instrument nach Anspruch 1, bei welchem in der Instrumentenhandhabe (1) eine mit dem beweglichen Griffteil (4) verbundene Aufnahme (8) zur Verbindung mit dem proximale Ende der Betätigungsstange (52) angeordnet ist, wobei die Blockiermittel ein an der Aufnahme (8) angeordnetes Sperrelement (14) aufweisen, das von einem Federelement (16) in eine blockierende Position und von dem proximalen Ende der Betätigungsstange (52) gegen die Federkraft des Federelementes (16) in eine gelöste Position bewegbar ist.

3. Medizinisches Instrument nach Anspruch 1 oder 2, bei welchem die Rastverbindung (12, 54) zwischen der Betätigungsstange (52) und dem beweglichen Griffteil (4) als Kugelrastverbindung ausgebildet ist.

4. Medizinisches Instrument nach Anspruch 3, bei welchem am proximalen Ende der Betätigungsstange (52) umfänglich eine Ringnut (54) ausgebildet ist und die Aufnahme als Zylinderhülse (8) ausgebildet ist, in welche das proximale Ende der Betätigungsstange (52) einsetzbar ist und in welcher zumindest eine Rastkugel (12) angeordnet ist, welche mit der Ringnut (54) in Eingriff treten kann.

5. Medizinisches Instrument nach Anspruch 4, bei welchem die zumindest eine Rastkugel (12) in einer Durchgangsbohrung in der Wandung der Zylinderhülse (8) in einer Richtung quer zur Längsachse (X) der Zylinderhülse (8) beweglich angeordnet ist, wobei die Rastkugel (12) einen Durchmesser aufweist, welcher größer als die Dicke der Wandung ist.

6. Medizinisches Instrument nach Anspruch 5, bei welchem an einer Führung (10), in der die Zylinderhülse (8) geführt ist, eine Ausnehmung oder Schulter (20) vorgesehen ist, mit welcher die Rastkugel (12) in Eingriff treten kann, um die Rastkugel (12) von der Betätigungsstange (52) außer Eingriff zu bringen und gleichzeitig die Zylinderhülse (8) in der Führung (10) zu blockieren.

7. Medizinisches Instrument nach Ansprüchen 2 und 6, bei welchem das Sperrelement (14) im Inneren der Zylinderhülse (8) angeordnet ist und in seiner blockierenden Position die Rastkugel (12) mit der Ausnehmung oder Schulter (20) in der Führung (10) in Eingriff hält und in seiner gelösten Position die Rastkugel (12) freigibt.

8. Medizinisches Instrument nach einem der vorangehenden Ansprüche, bei welchem der Instrumenteneinsatz (42) einen Instrumentenschaft (46) aufweist, dessen proximales Ende als Lueranschluss (51) ausgebildet ist.

9. Medizinisches Instrument nach einem der vorangehenden Ansprüche, bei welchem der Instrumenteneinsatz (42) einen Instrumentenschaft (46) aufweist, welcher lösbar mit der Instrumentenhandhabe (1) über eine Rastverbindung (50, 40; 58) verbindbar ist.

10. Medizinisches Instrument nach Anspruch 8, bei welchem die Rastverbindung einen an der Instrumentenhandhabe angeordneten, quer zur Längsachse des Instrumentenschaftes bewegbaren Rastbolzen (58) aufweist, welcher mit einer Ausnehmung am proximalen Ende des Instrumentenschaftes (46) lösbar in Eingriff treten kann.

11. Medizinisches Instrument nach Ansprüchen 8 und 10, bei welchem der Rastbolzen (58) den Lueranschluss (51) hintergreift.

12. Medizinisches Instrument nach Anspruch 9, bei welchem die Rastverbindung zwischen Instrumentenschaft (46) und Instrumentenhandhabe (1) als Kugelrastverbindung (50, 40) ausgebildet ist.

13. Medizinisches Instrument nach Anspruch 12, bei welchem die Kugelrastverbindung (50, 40) zwischen Instrumentenschaft (46) und Instrumentenhandhabe (1) ein Verschlusselement (22) zur Sicherung der Kugelrastverbindung (50, 40) und ein Griffelement (24) aufweist, wobei das Verschlusselement (22) sowohl durch Bewegung des Griffelementes (24) als auch unabhängig von der Bewegung des Griffelementes (24) in eine entsicherte Position bewegbar ist.

14. Medizinisches Instrument nach Anspruch 12 oder 13, bei welchem die Kugelrastverbindung (50, 40) zwischen Instrumentenschaft (46) und Instrumentenhandhabe (1) eine Verschlusshülse (22) zur Sicherung der Kugelrastverbindung (50, 40) und eine Griffhülse (24) aufweist, wobei die Verschlusshülse (22) in einer gesicherten Position die Bewegung zumindest einer Rastkugel (50) blockiert und in einer entsicherten Position die Rastkugel (50) freigibt, und die Verschlusshülse (22) sowohl durch Bewegung der Griffhülse (24) als auch unabhängig von einer Bewegung der Griffhülse (24) in die entsicherte Position bewegbar ist.

15. Medizinisches Instrument nach Anspruch 14, bei welchem die Verschlusshülse (22) im Inneren der Griffhülse (24) angeordnet ist und durch ein Federelement (30) in der gesicherten Position gehalten wird, wobei die Verschlusshülse (22) zumindest eine Anlageschulter (32) aufweist, welche von einer Anlageschulter (34) der Griffhülse (24) nur in einer der Federkraft des Federelementes (30) entgegengesetzten Richtung hintergriffen wird.

16. Medizinisches Instrument nach Anspruch 15, bei welchem die Verschlusshülse (22) und die Griffhülse (24) an der Instrumentenhandhabe (1) beweglich geführt sind und die Griffhülse (24), wenn die Verschlusshülse (22) in ihrer gesicherten Position ist, an einem Anschlag (36) an der Instrumentenhandhabe (1) zur Anlage kommt.

## Claims

1. A medical instrument with an instrument insert (42) and with an instrument handle (1) which are detachably connectable to one another, wherein an actuation rod (52) of the instrument insert (42) is detachably connectable to a movable grip part (4) of the instrument handle (1) via a locking connection (12, 54), and the medical instrument comprises blocking means (14) are provided which block the movable grip part (4) given a detached actuation rod (52).

2. A medical instrument according to claim 1, with which a receiver (8) connected to the movable grip part (4), for connection to the proximal end of the actuation rod (52), is arranged in the instrument handle (1), wherein the blocking means comprise a blocking element (14) which is arranged on the receiver (8) and which may be moved by a spring element (16) into a blocking position, and by the proximal end of the actuation rod (52) against the spring force of the spring element (16) into a released position.

3. A medical instrument according to claim 1 and 2, with which the locking connection (12, 54) is designed as a ball locking connection between the actuation rod (52) and the movable grip part (4).

4. A medical instrument according to claim 3, with which an annular groove (54) is formed peripherally at the proximal end of the actuation rod (52), and the receiver is designed as a cylinder sleeve (8) into which the proximal end of the actuation rod (52) may be inserted and in which at least one locking ball (12) able to engage with the annular groove (54) is arranged.

5. A medical instrument according to claim 4, with which the at least one locking ball (12) is movably arranged in a through-bore in the wall of the cylinder sleeve (8) in a direction transverse to the longitudinal axis (X) of the cylinder sleeve (8), wherein the locking ball (12) has a diameter which is larger than the thickness of the wall.

6. A medical instrument according to claim 5, with which a recess or shoulder (20) is provided on a guide (10) in which the cylinder sleeve (8) is guided, and the locking ball (12) may engage with said recess or shoulder in order to disengage the locking ball (12) from the actuation rod (52) and to simultaneously block the cylinder sleeve (8) in the guide (10).

7. A medical instrument according to claims 2 and 6, with which the blocking element (14) is arranged in the inside of the cylinder sleeve (8) and in its blocking position holds the locking ball (12) engaged with the recess or shoulder (20) in the guide (10) and in its released position releases the locking ball (12).

8. A medical instrument according to of the preceding claims, with which the instrument insert (42) comprises an instrument shank (46) whose proximal end is designed as a Luer connection (51).

9. A medical instrument according to one of the preceding claims, with which the instrument insert (42) comprises an instrument shank (46) which is detachably connectable to the instrument handle (1) via a locking connection (50, 40; 58).

10. A medical instrument according to claim 8, with which the locking connection comprises a locking bolt (58) which is arranged on the instrument handle, is movable transversely to the longitudinal axis of the instrument shank, and may releasably engage with a recess at the proximal end of the instrument shank (46).

11. A medical instrument according to one of the claims 8 and 10, with which the locking bolt (58) engages behind the Luer connection (51).

12. A medical instrument according to claim 9, with which the locking connection between the instrument shank (46) and the instrument handle (1) is designed as a ball locking connection (50, 40).

13. A medial instrument according to claim 12, with which the ball locking connection (50, 40) between the instrument shank (46) and the instrument handle (1) comprises a closure element (22) for securing the ball locking connection (50, 40), and a grip element (24), wherein the closure element (22) may be moved into an unsecured position by way of movement of the grip element (24) as well as independently of the movement of the grip element (24).

14. A medical instrument according to claim 12 or 13, with which the ball locking connection (50, 40) between the instrument shank (46) and the instrument handle (1) comprises a closure sleeve (22) for securing the ball locking connection (50, 40), and a grip sleeve (24), wherein the closure sleeve (22) in a secured position blocks the movement at least of one locking ball (50), and in an unsecured position releases the locking ball (50), and the closure sleeve (22) may be moved into the unsecured position by way of movement of the grip sleeve (24) as well as independently of a movement of the grip sleeve (24).

15. A medical instrument according to claim 14, with which the closure sleeve (22) is arranged in the inside of the grip sleeve (24) and is held in the secure position by way of a spring element (30), wherein the closure sleeve (22) comprises at least one contact shoulder (32) behind which a contact shoulder (34) of the grip sleeve (24) engages only in a direction opposite to the spring force of the spring element (30).

16. A medical instrument according to claim 15, with which the closure sleeve (22) and the grip sleeve (24) are movably guided on the instrument handle (1), and the grip sleeve (24), when the closure sleeve (22) is in its secured position, comes to bear on an abutment (36) on the instrument handle (1).

## Revendications

1. Instrument à usage médical comportant un insert d'instrument (42) et une poignée d'instrument (1), qui sont assemblés l'un à l'autre d'une manière démontable, une tige de commande (52) de l'insert d'instrument (42) pouvant, par l'intermédiaire d'un assemblage par encliquetage (12, 54), être assemblée d'une manière amovible à une partie formant manche mobile (4) de la poignée d'instrument (1), et l'instrument à usage médical comprenant des moyens de blocage (14), qui, quand la tige de commande (52) a été démontée, bloquent la partie formant manche mobile (4).

2. Instrument à usage médical selon la revendication 1, dans lequel un logement (8), relié à la partie formant manche mobile (4), est aménagé dans la poignée d'instrument (1) pour se raccorder à l'extrémité proximale de la tige de commande (52), les moyens de blocage comportant un élément d'arrêt (14), disposé contre le logement (8), l'élément d'arrêt pouvant être déplacé par un élément de ressort (16) dans une position de blocage, et par l'extrémité proximale de la tige de commande (52) contre la force de ressort de l'élément de ressort (16) dans une position démontée.

3. Instrument à usage médical selon la revendication 1 ou 2, dans lequel l'assemblage par encliquetage (12, 54), entre la tige de commande (52) et la partie formant manche mobile (4), est configuré sous forme d'un assemblage par encliquetage à bille d'arrêt.

4. Instrument à usage médical selon la revendication 3, dans lequel une rainure annulaire (54) est formée sur la périphérie de l'extrémité proximale de la tige de commande (52), et le logement est configuré sous forme d'une douille cylindrique (8), dans laquelle l'extrémité proximale de la tige de commande (52) peut être insérée, et dans laquelle au moins une bille d'arrêt (12) est disposée, qui peut s'engrener avec la rainure annulaire (54).

5. Instrument à usage médical selon la revendication 4, dans lequel ladite au moins une bille d'arrêt (12) est disposée d'une manière mobile dans un trou traversant aménagé dans la paroi de la douille cylindrique (8), dans une direction perpendiculaire à l'axe longitudinal (X) de la douille cylindrique (8), la bille d'arrêt (12) ayant un diamètre supérieur à l'épaisseur de la paroi.

6. Instrument à usage médical selon la revendication 5, dans lequel il est prévu, contre une glissière de guidage (10) dans laquelle est guidée la douille cylindrique (8), un évidement ou un épaulement (20), avec lequel la bille d'arrêt (12) peut entrer en prise, pour désengrener la bille d'arrêt (12) d'avec la tige de commande (52) et simultanément bloquer la douille cylindrique (8) dans la glissière de guidage (10).

7. Instrument à usage médical selon les revendications 2 et 6, dans lequel l'élément d'arrêt (14) est disposé à l'intérieur de la douille cylindrique (8) et, dans sa position bloquante, va maintenir la bille d'arrêt (12), avec l'évidement ou l'épaulement (20), engrenée dans la glissière de guidage (10), et en position démontée libère la bille d'arrêt (12).

8. Instrument à usage médical selon l'une des revendications précédentes, dans lequel l'insert d'instrument (42) comprend une tige d'instrument (46), dont l'extrémité proximale est configurée sous forme d'un embout Luer (51).

9. Instrument à usage médical selon l'une des revendications précédentes, dans lequel l'insert d'instrument (42) comprend une tige d'instrument (46) qui est reliée d'une manière démontable à la poignée d'instrument (1) par l'intermédiaire d'un assemblage par encliquetage (50, 40; 58).

10. Instrument à usage médical selon la revendication 8, dans lequel l'assemblage par encliquetage comprend un boulon d'encliquetage (58), disposé contre la poignée d'instrument et mobile perpendiculairement à l'axe longitudinal de la tige de l'instrument, boulon qui peut s'engrener de manière démontable avec un évidement aménagé à l'extrémité proximale de la tige d'instrument (46).

11. Instrument à usage médical selon les revendications 8 et 10, dans lequel le boulon d'encliquetage (58) s'engage derrière l'embout Luer (51).

12. Instrument à usage médical selon la revendication 9, dans lequel l'assemblage par encliquetage entre la tige d'instrument (46) et la poignée d'instrument (1) est configuré sous forme d'un assemblage par encliquetage à bille d'arrêt (50, 40).

13. Instrument à usage médical selon la revendication 12, dans lequel l'assemblage par encliquetage à bille d'arrêt (50, 40) comprend, entre la tige d'instrument (46) et la poignée d'instrument (1), un élément de verrouillage (22) pour bloquer l'assemblage par encliquetage à bille d'arrêt (50, 40) et un élément de préhension (24), l'élément de verrouillage (22) étant mobile, pour parvenir dans une position débloquée, tant en déplaçant l'élément de préhension (24), qu'indépendamment du déplacement de l'élément de préhension (24).

14. Instrument à usage médical selon la revendication 12 ou 13, dans lequel l'assemblage par encliquetage à bille d'arrêt (50, 40) comprend, entre la tige d'instrument (46) et la poignée d'instrument (1), une douille de verrouillage (22) pour bloquer l'assemblage par encliquetage à bille d'arrêt (50, 40), ainsi qu'une douille de préhension (24), la douille de verrouillage (22), dans une position bloquée, bloquant le déplacement d'au moins une bille d'arrêt (50) et, dans une position débloquée, libérant la bille d'arrêt (50), et la douille de verrouillage (22) étant mobile, pour parvenir dans une position débloquée, tant en déplaçant la douille de préhension (24) qu'indépendamment d'un déplacement de la douille de préhension (24).

15. Instrument à usage médical selon la revendication 14, dans lequel la douille de verrouillage (22) est disposée à l'intérieur de la douille de préhension (24) et est maintenue en position bloquée par un élément de ressort (30), la douille de verrouillage (22) comportant au moins un épaulement d'appui (32), qui est engagé par l'arrière par un épaulement d'appui (34) de la douille de préhension (24), seulement dans une direction opposée à la force de ressort de l'élément de ressort (30).

16. Instrument à usage médical selon la revendication 15, dans lequel la douille de verrouillage (22) et la douille de préhension (24) sont guidées d'une manière mobile contre la poignée d'instrument (1), et la douille de préhension (24), quand la douille de verrouillage (22) se trouve dans sa position bloquée, vient s'appuyer contre une butée (36) disposée au niveau de la poignée d'instrument (1).
